# NEW EUROPEAN PATENT SPECIFICATION

(11) **EP 1 791 495 B2**
(45) Date of publication and mention of the opposition decision: **01.06.2022**
(45) Mention of the grant of the patent: 22.04.2015
(21) Application number: 05792374.0
(22) Date of filing: 31.08.2005
(51) Int. Cl.: A61F 2/95, A61F 2/966, A61F 2/01, A61F 2/24, A61F 2/958

(54) **DELIVERY SYSTEM WHICH FACILITATES HYDRATION OF AN INTRALUMINAL MEDICAL DEVICE**
ABGABESYSTEM ZUR ERLEICHTERUNG DER HYDRATATION EINES INTRALUMINALEN MEDIZINPRODUKTS
SYSTEME D'APPORT QUI FACILITE L'HYDRATATION D'UN DISPOSITIF MEDICAL INTRALUMINAL

(30) Priority: 01.09.2004 US 606349 P
(43) Date of publication of application: 06.06.2007
(73) Proprietor: Cook Medical Technologies LLC, Bloomington, IN 47404 (US)
(72) Inventor: CASE, Brian, C., Bloomington, IN 47404 (US); FLAGLE, Jacob, A., New Palestine, IN 46163 (US); PAUL, Ram, H., Jr., Bloomington, IN 47403 (US); SCHAEFFER, Darin, G., Bloomington, IN 47403 (US)
(74) Representative: Mathys & Squire
(86) International application number: PCT/US2005/030861
(87) International publication number: WO 2006/028821

(56) References cited:
- WO-A-94/15549
- WO-A-03/045275
- WO-A2-98/23241
- US-A- 5 534 007
- US-A- 5 776 140
- US-A1- 2001 044 648
- US-A1- 2003 125 791
- US-B1- 6 168 617

## Description

### FIELD OF THE INVENTION

The present invention relates to medical devices. More particularly, the invention relates to a delivery system for implantation of an intraluminal medical device in a body vessel.

### BACKGROUND

Minimally invasive techniques and instruments for placement of intraluminal medical devices have been developed over recent years and are frequently used to deliver and deploy an intraluminal medical device at a desired point of treatment. In these techniques, a delivery system is used to carry the intraluminal medical device through a body vessel to the point of treatment. Once the point of treatment is reached, the intraluminal medical device is deployed from the delivery system. The delivery system is subsequently withdrawn from the point of treatment and, ultimately, the body vessel. A wide variety of treatment devices that utilize minimally invasive technology has been developed and includes stents, stent grafts, occlusion devices, infusion catheters, prosthetic valves, and the like.

Some intraluminal medical devices include a portion that may require hydration or that can be hydrated. For example, some prosthetic valves, include a graft member formed of a material, such as an extracellular matrix (ECM) material, that can be hydrated prior to implantation. It may be desirable to accomplish the hydration of the intraluminal medical device while the device is located in the delivery system and prior to or during implantation of the device at the desired point of treatment in the body vessel. Reference is directed to WO 03/045275 which discloses a delivery system for implanting a medical device such as a venous valve. The system comprises a catheter and an inner member with a flat portion on the inner member serving with the catheter to form a passageway to transmit hydrating fluid to the valve prior to implantation.

There exists a need for a delivery system which facilitates the hydration of the intraluminal medical device while the intraluminal medical device is positioned in the delivery system.

### SUMMARY OF EXEMPLARY EMBODIMENTS OF THE INVENTION

The invention provides delivery systems for delivering an intraluminal medical device to a point of treatment in a body vessel. Delivery systems according to the invention facilitate the hydration of the intraluminal medical device while the intraluminal medical device is positioned in the delivery system. The scope of the present invention is set forth in the appended claims.

In one example, a delivery system comprises an elongate tubular member having a distal end adapted for insertion into a body vessel. A dilator is disposed in the tubular member. The dilator has a lumen formed in an axial direction therein and has a distal end adapted for insertion into the body vessel. The tubular member and the dilator have a device chamber formed therebetween. An intraluminal medical device is disposed in the device chamber. A hydration channel is formed by at least one of the tubular member and the dilator to facilitate hydration of the intraluminal medical device.

In another example, a delivery system comprises an elongate tubular member having a distal end adapted for insertion into a body vessel. A dilator is disposed in said tubular member. The dilator has a lumen formed in an axial direction therein and a distal end adapted for insertion into the body vessel. The tubular member and the dilator have a device chamber formed therebetween. An intraluminal medical device is disposed in the device chamber. A hydration channel is formed by the dilator to facilitate hydration of the intraluminal medical device.

In another example, a delivery system comprises an elongate tubular member having a distal end adapted for insertion into a body vessel. A dilator is disposed in said tubular member. The dilator has a lumen formed in an axial direction therein and a distal end adapted for insertion into the body vessel. The tubular member and the dilator have a device chamber formed therebetween. A hydration channel is formed by the tubular member to facilitate hydration of the intraluminal medical device.

There are also described methods of hydrating an intraluminal medical device.

One exemplary method comprises the steps of providing a delivery system having an intraluminal medical device disposed therein and a hydration channel formed therein. The hydration channel is in fluid communication with the intraluminal medical device and a source of hydrating fluid. A hydrating fluid is introduced into the hydration channel. The hydrating fluid is caused to flow through the hydrating channel to communicate with the intraluminal medical device to provide hydration thereof.

Another exemplary method comprises the steps of providing a delivery system comprising an elongate tubular member having a distal end adapted for insertion into a body vessel; a dilator disposed in the tubular member, the dilator having a lumen formed in an axial direction therein and a distal end adapted for insertion into the body vessel, the tubular member and the dilator having a device chamber formed therebetween; an intraluminal medical device disposed in the device chamber; and a hydration channel formed by at least one of the tubular member and the dilator, the hydration channel in fluid communication with the device chamber and a source of hydrating fluid. In another step of the method, a hydrating fluid is introduced into the hydration channel. The hydrating fluid is caused to flow through the hydrating channel to communicate with the device chamber to provide hydration of the intraluminal medical device.

Additional understanding of the invention can be obtained with the review of the description of exemplary embodiments, appearing below, and the appended drawings that illustrate exemplary embodiments.

### BRIEF DESCRIPTION OF THE DRAWINGS

Figure 1 is a perspective view of a delivery system.
Figure 2 is a sectional view of the distal end of a prior art delivery system.
Figure 3 is a sectional view of the distal end of the delivery system illustrated in Figure 1.
Figure 4 is a sectional view of the distal end of the delivery system illustrated in Figures 1 and 3 taken along line 4-4.
Figure 5 is a sectional view of an alternate embodiment of the delivery system illustrated in Figure 3.
Figure 6 is a sectional view of the distal end of the delivery system illustrated in Figure 5 taken along line 6-6.
Figure 7 is a sectional view of an alternate embodiment of the delivery system illustrated in Figure 3.
Figure 8 is a sectional view of the distal end of the delivery system illustrated in Figure 7 taken along line 8-8.
Figure 9 is a sectional view of an alternate embodiment of the delivery system illustrated in Figure 3.
Figure 10 is a sectional view of the distal end of the delivery system illustrated in Figure 9 taken along line 10-10.
Figure 11 is a sectional view of an alternate embodiment of the delivery system illustrated in Figure 3.
Figure 12 is a sectional view of the distal end of the delivery system illustrated in Figure 11 taken along line 12-12.
Figure 13 is a sectional view of an alternate embodiment of the delivery system illustrated in Figure 3.
Figure 14 is a sectional view of the distal end of the delivery system illustrated in Figure 13 taken along line 14-14.
Figure 15 is a sectional view of an alternate embodiment of the delivery system illustrated in Figure 3.
Figure 16 is a sectional view of the distal end of the delivery system illustrated in Figure 15 taken along line 16-16.
Figure 17 is a sectional view of an alternate embodiment of the delivery system illustrated in Figure 3.
Figure 18 is a sectional view of a body vessel containing an alternate embodiment of the delivery system illustrated in Figure 3.
Figure 19 is a sectional view of an alternate embodiment of the delivery system illustrated in Figure 3.
Figure 20 is a perspective view of the distal end of the delivery system illustrated in Figure 19.
Figure 21 is a sectional view of an alternate example of the delivery system illustrated in Figure 3.

### DETAILED DESCRIPTION OF EXEMPLARY EMBODIMENTS OF THE INVENTION

The following detailed description and appended drawings describe and illustrate various exemplary embodiments of the invention. The description and drawings serve to enable one skilled in the art to make and use the invention, and are not intended to limit the scope of the invention, or its protection, in any manner.

As used herein, the term "hydrate," and grammatically related terminology, refers to the exposure of a surface of an item, such as an intraluminal medical device, to a fluid, such as a liquid. The term does not require any particular fluid or fluid type, and does not require any retention of the fluid by the surface of the item or by the item as a whole.

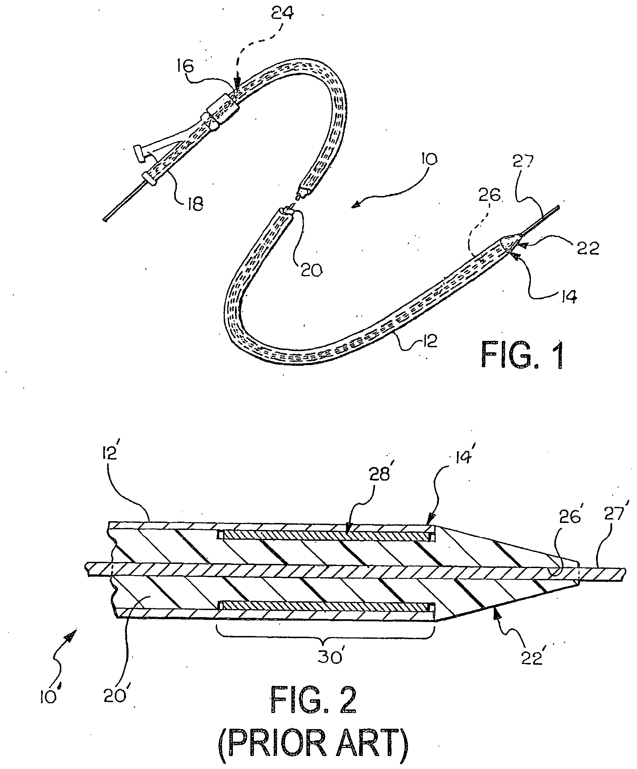

Figure 1 illustrates a delivery system 10. The delivery system 10 includes an elongate sheath or tubular member 12 having a distal end 14 which is insertable in a body vessel and a proximal end 16 that can be coupled to a connector 18 such as a Touhy Borst adapter, for example. The tubular member 12 is formed of a flexible material, such as polyurethane or other suitable polymeric material, for example.

The delivery system 10 includes a dilator 20 disposed within the tubular member 12. As used herein, the term "dilator" refers to an elongate member capable of being disposed within a lumen of a sheath, such as the tubular member 12. The dilator 20 has a tapered distal end 22, which is insertable in the body vessel, and a proximal end 24. A lumen 26 is formed by the dilator 20 and extends along the entire length of the dilator 20. The lumen 26 is adapted to receive a wireguide 27, or any other suitable member, therein. As used herein, the term "wireguide" refers to elongate members used in minimally invasive procedures to define a path along which other devices can be advanced. The term is considered equivalent in meaning to the term "guidewire" as also used in the art. The lumen 26 may aid in guiding the delivery system 10 through the body vessel to a desired point of treatment.

Figure 2 illustrates the distal end 22 ' of a prior art delivery system 10'. Structure repeated from Figure 1 is represented by the same reference numeral and a prime (') symbol. An intraluminal medical device 28' is shown disposed in a device chamber 30' formed in the dilator 20' adjacent the distal end 22 '. The intraluminal medical device 28' may be any suitable intraluminal medical device, examples of which include a stent, a prosthetic valve, a filter, an occlusion device, a distal protection device, a stent graft, and the like.

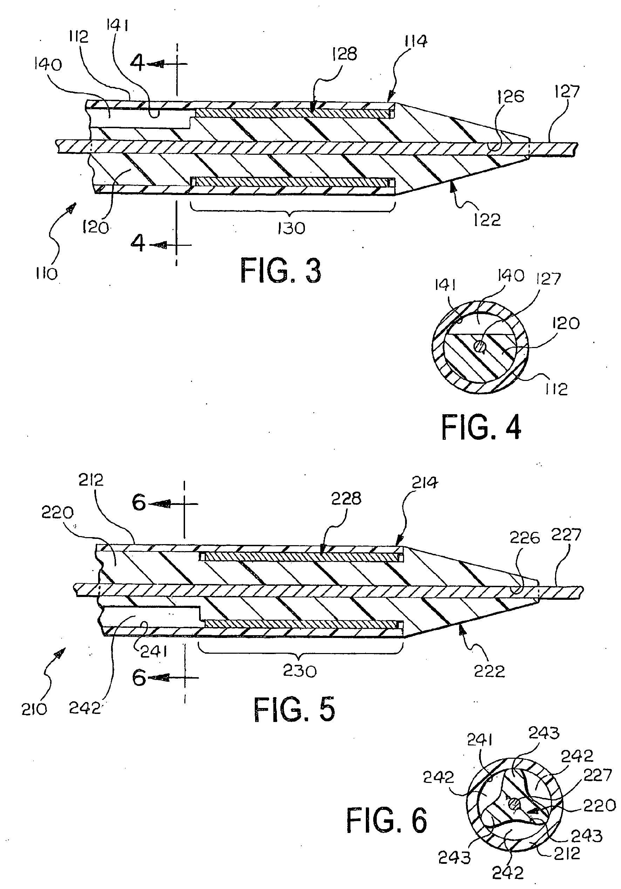

Figure 3 shows the distal end 122 of the delivery system 110 illustrated in Figure 1 according to another example of the invention. Repeated structure is represented by the same two digit reference numerals from Figure 1 preceded by a 1. For example, the delivery system is designated by the reference numeral 110. In the embodiment shown, the dilator 120 is formed to create a space or hydration channel 140 between the dilator 120 and an inner wall 141 of the tubular member 112. The hydration channel 140 can extend substantially along the entire length of the dilator 120 to provide fluid communication between the proximal end (not illustrated in Figure 3) of the dilator 120 and the device chamber 130 at the distal end 122 of the dilator 120. Alternatively, the hydration channel 140 can extend along any desired and suitable length of the dilator 120.
The specific length chosen for the hydration channel in a particular delivery device according to the invention will depend on several considerations, including the desired mechanism for hydrating the intraluminal medical device. The cross- sectional shape of the dilator 120 is substantially D-shaped as illustrated in Figure 4 to create the hydration channel 140. The hydration channel 140 is shown at an upper portion of the tubular member 112. However, it is understood that the hydration channel 140 can be placed in any position within the tubular member 112. Any conventional method such as extruding the dilator 120, compression of an outer surface of the dilator 120, mechanical removal of material from the dilator 120, and other methods, can be used to form the dilator 120 in the desired shape to create the hydration channel 140. As used herein, the term "channel" refers to a structure that allows fluid flow from one point to another. Non-limiting examples of channels include a conduit, an aperture, a hole, a chamber, a gap, a space, a vacant area, and the like.

Figure 5 shows the distal end 222 of the delivery system 210 illustrated in Figure 1 according to another example. Repeated structure has the same last two digits of the reference numerals from the previous figures, preceded by a 2. For example, the delivery system is designated by the reference numeral 210. In the embodiment shown, the dilator 220 is formed to create three spaced apart gaps or hydration channels 242 between the dilator 220 and an inner wall 241 of the tubular member 212. The hydration channels 242 can extend substantially along the entire length of the dilator 220 to provide fluid communication between the proximal end 224 (not illustrated in Figure 5) of the dilator 220 and the device chamber 230 at the distal end 222 of the dilator 220, or along any other suitable length of the dilator. The cross-sectional shape of the dilator 220 includes three radially outwardly extending lobes 243 as illustrated in Figure 6 which extend to abut the inner wall 241 of the tubular member 212. Thus, the dilator 220 is centered in the tubular member 212. It is understood that more or fewer lobes 243 can be used as desired. The hydration channels 242 are formed between adjacent lobes 243. It is understood that one or more of the lobes 243 can be formed to not abut the inner wall 241 of the tubular member 212 allowing fluid communication between two or more adjacent hydration channels 242. Any conventional method such as extruding the dilator 220, compression of an outer surface of the dilator 220, mechanical removal of material from the dilator 220, and other methods, can be used to form the dilator 220 in the desired shape to create the hydration channels 242.

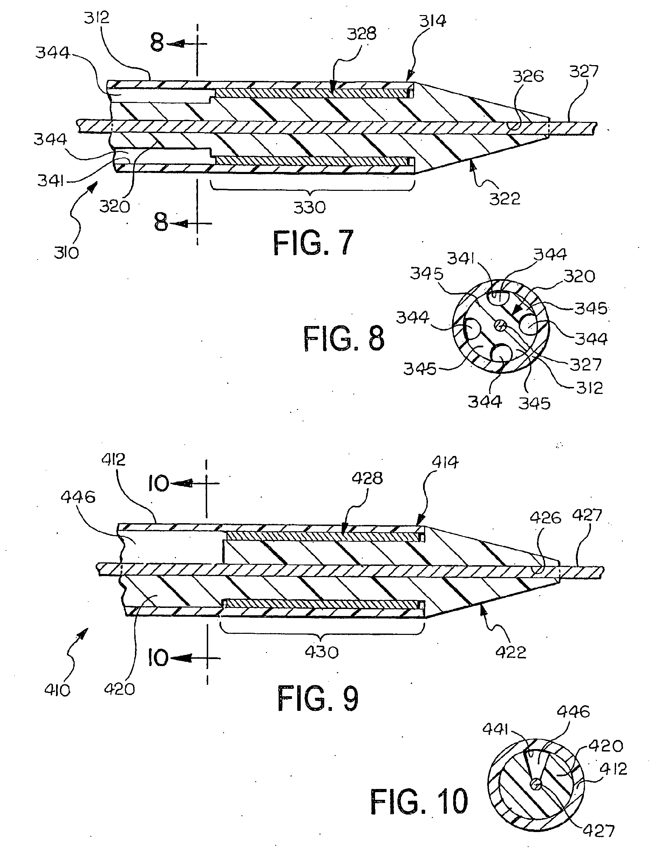

Figure 7 shows the distal end 322 of the delivery system 310 illustrated in Figure 1 according to another example. Repeated structure has the same last two digits of the reference numerals from the previous figures, preceded by a 3. For example, the delivery system is designated by the reference numeral 310. In the embodiment shown, the dilator 320 is formed to create four spaced apart gaps or hydration channels 344 between the dilator 320 and an inner wall 341 of the tubular member 312. The hydration channels 344 can extend substantially along the entire length of the dilator 320 to provide fluid communication between the proximal end 324 (not illustrated in Figure 7) of the dilator 320 and the device chamber 330 at the distal end 322 of the dilator 320 or along any other suitable length of the dilator. The cross-sectional shape of the dilator 320 includes four radially outwardly extending lobes 345 as illustrated in Figure 8 which extend to abut the inner wall 341 of the tubular member 312. Thus, the dilator 320 is centered in the tubular member 312. It is understood that more or fewer lobes 345 can be used as desired. The hydration channels 344 are formed between adjacent lobes 345. It is understood that one or more of the lobes 345 can be formed to not abut the inner wall 341 of the tubular member 312 allowing fluid communication between two or more adjacent hydration channels 344. Any conventional method such as extruding the dilator 320, compression of an outer surface of the dilator 320, mechanical removal of material from the dilator 320, and other methods, can be used to form the dilator 320 in the desired shape to create the hydration channels 344.

In Figure 9, the distal end 422 is shown of the delivery system 410 illustrated in Figure 1 according to another example. Repeated structure has the same last two digits of the reference numerals from the previous figures, preceded by a 4. For example, the delivery system is designated by the reference numeral 410. In the embodiment shown, the dilator 420 is formed to create a space or hydration channel 446 between the dilator 420 and an inner wall 441 of the tubular member 412. The hydration channel 446 can extend substantially along the entire length of the dilator 420 to provide fluid communication between the proximal end 424 (not illustrated in Figure 9) of the dilator 420 and the device chamber 430 at the distal end 422 of the dilator 420 or along any other suitable length of the dilator. The cross-sectional shape of the dilator 420 is substantially C-shaped with the open part of the C facing upwardly as illustrated in Figure 10 to form the hydration channel 446. It is understood that the open portion of the C forming the hydration channel 446 can be placed in any position within the tubular member 412 or can face in any direction. Any conventional method such as extruding the dilator 420, cutting a radial portion of the dilator 420, other mechanical removal of material from the dilator 420, and other methods, can be used to form the dilator 420 in the desired shape to create the hydration channel 446. The wireguide 427 is disposed in the central portion of the C-shaped cross-section of the dilator 420. In this embodiment, the lumen 426 which receives the wireguide 427 therein, is in fluid communication with the hydration channel 446.

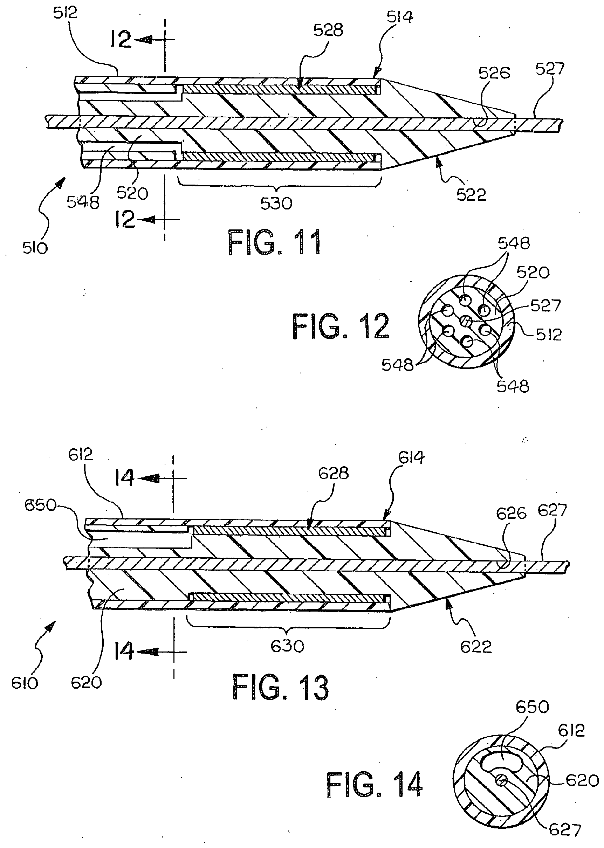

Figure 11 shows the distal end 522 of the delivery system 510 illustrated in Figure 1 according to another example of the invention. Repeated structure has the same last two digits of the reference numerals from the previous figures, preceded by a 5. For example, the delivery system is designated by the reference numeral 510. The dilator 520 includes an annular array of axially extending hydration channels 548 formed therein. In the embodiment shown in Figure 12, six hydration channels 548 are formed in the dilator 520. However, it is understood that more or fewer hydration channels 548 can be used as desired. In the illustrated embodiment, each of the hydration channels 548 are spaced equidistant from at least two other hydration channels and from the lumen 526 that receives the wireguide 527. It is understood, though, that any desired and suitable spacing of hydration channels 548 can be used. The hydration channels 548 can extend substantially along the entire length of the dilator 520 to provide fluid communication between the proximal end 524 (not illustrated in Figure 11) of the dilator 520 and the device chamber 530 at the distal end 522 of the dilator 520 or along any other suitable length of the dilator. Any conventional method such as extruding the dilator 520, mechanical removal of material from the dilator 520, and other methods, can be used to form the hydration channels 548 in the dilator 520.

Figure 13 shows the distal end 622 of the delivery system 610 illustrated in Figure 1 according to another example of the invention. Repeated structure has the same last two digits of the reference numerals from the previous figures, preceded by a 6. For example, the delivery system is designated by the reference numeral 610. The dilator 620 includes an axially extending passage or hydration channel 650 formed therein. It is understood that more hydration channels 650 can be used as desired. The hydration channel 650 has a kidney cross-sectional shape as illustrated in Figure 14. It is understood that other cross-sectional shapes can be used as desired. Additionally, the hydration channel 650 is shown formed in an upper portion of the dilator 620. It is understood that the hydration channel 650 can be formed in any portion of the dilator 620 as desired. The hydration channel 650 can extend substantially along the entire length of the dilator 620 to provide fluid communication between the proximal end 624 (not illustrated in Figure 13) of the dilator 620 and the device chamber 630 at the distal end 622 of the dilator 620, or along any other suitable length of the dilator. Any conventional method such as extruding the dilator 620, mechanical removal of material from the dilator 620, and other methods, can be used to form the hydration channel 650 in the dilator 620.

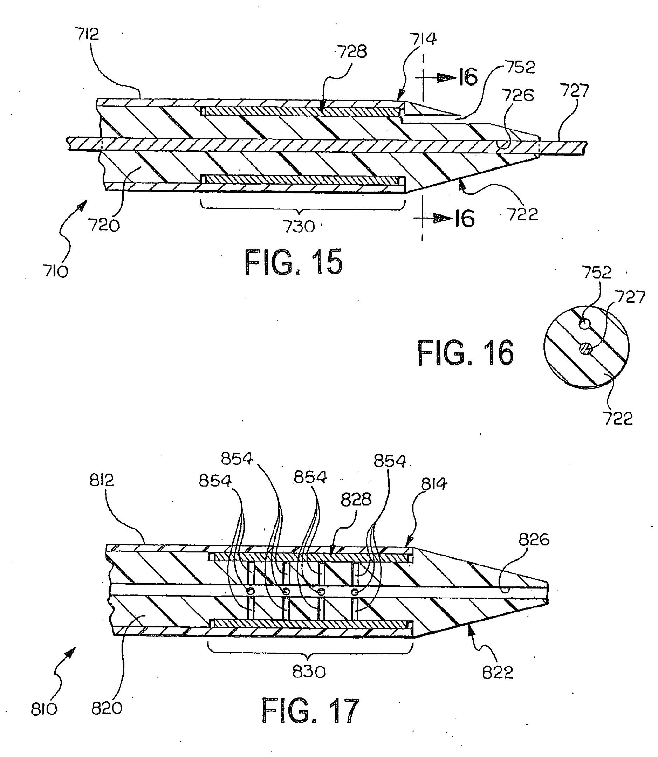

In Figure 15, the distal end 722 is shown of the delivery system 710 illustrated in Figure 1 according to another example of the invention. Repeated structure has the same last two digits of the reference numerals from the previous figures, preceded by a 7. For example, the delivery system is designated by the reference numeral 710. The distal end 722 of the dilator 720 includes a hydration channel 752 formed therein. It is understood that more hydration channels 752 can be used as desired. The hydration channel 752 extends proximally from the distal end 722 of the dilator 720 to the device chamber 730 to provide fluid communication between the distal end 722 and the device chamber 730. The hydration channel 752 is shown with a circular cross- sectional shape as illustrated in Figure 16. It is understood that other cross- sectional shapes can be used as desired. The hydration channel 752 can also be formed as an open channel in the distal end 722 having a U-shaped cross-section or other shape as desired. Additionally, the hydration channel 752 is shown formed in an upper portion of the dilator 720. It is understood that the hydration channel 752 can be formed in any portion of the dilator 720 as desired. Any conventional method such as extruding the dilator 720, mechanical removal of material from the dilator 720, and other methods, can be used to form the hydration channel 752 in the dilator 720.

Figure 17 shows the distal end 822 of the delivery system 810 illustrated in Figure 1 according to an exemplary embodiment of the invention. Repeated structure has the same last two digits of the reference numerals from the previous figures, preceded by an 8. For example, the delivery system is designated by the reference numeral 810. The dilator 820 includes a plurality of holes or hydration channels 854 formed therein. The hydration channels 854 extend radially outwardly from the lumen 826 formed in the dilator 820 to the device chamber 830 to provide fluid communication between the device chamber 830 and the lumen 826, and eventually the distal end 822 or the proximal end 824 (not illustrated in Figure 17) of the dilator 820 as desired. It is understood that more or fewer hydration channels 854 can be used as desired. Although the plurality of hydration channels 854 are shown as a plurality of annular arrays, other patterns of the hydration channels 854 can be used, such as a helical pattern, for example, without departing from the scope of the invention. Any conventional method such as extruding the dilator 520, mechanical removal of material from the dilator 520, and other methods, can be used to form the hydration channels 548 in the dilator 520.

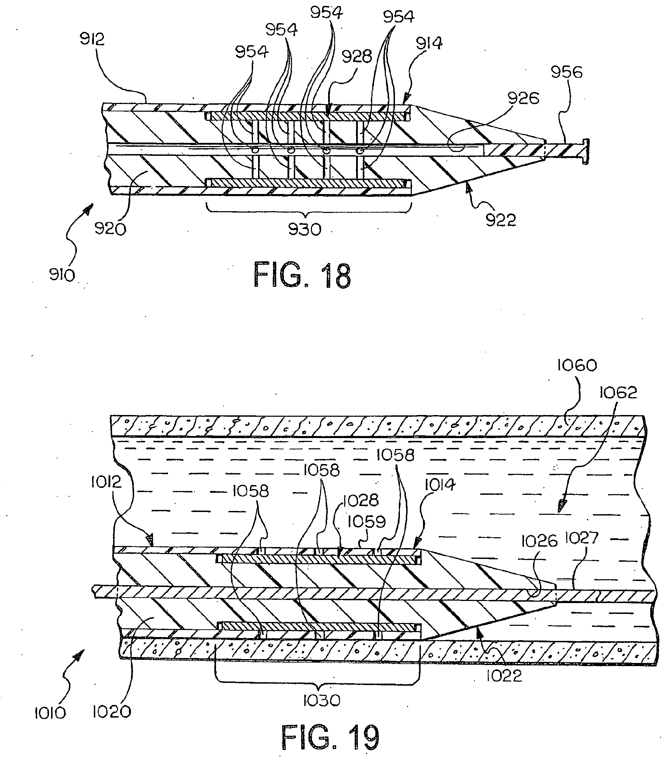

Figure 18 shows the distal end 922 of the delivery system 910 illustrated in Figure 1 according to another exemplary embodiment of the invention. Repeated structure has the same last two digits of the reference numerals from the previous figures, preceded by a 9. For example, the delivery system is designated by the reference numeral 910. The dilator 920 includes a plurality of hydration channels 954 formed therein. The hydration channels 954 extend radially outwardly from the lumen 926 formed in the dilator 920 to the device chamber 930 to provide fluid communication between the device chamber 930 and the lumen 926, and eventually the proximal end 924 (not illustrated in Figure 18) of the dilator 920. It is understood that more or fewer hydration channels 954 can be used as desired. Although the plurality of hydration channels 954 are shown as a plurality of annular arrays, other patterns of the hydration channels 954 can be used, such as a helical pattern, for example, without departing from the scope and spirit of the invention. Any conventional method such as extruding the dilator 920, mechanical removal of material from the dilator 920, and other methods, can be used to form the hydration channels 954 in the dilator 920. A removable plug 956 is disposed in the lumen 926 at the distal end 922 of the dilator 920. The plug 956 can be held in the lumen by any conventional means such as press fit, for example. It is desirable, but not critical, that the plug 956 sealingly close the lumen 926 at the distal end 922 of the dilator 920 to provide a substantially fluid tight seal. The plug 956 provides a temporary barrier to fluid egress from the lumen 926, and can be used to prevent such egress during a hydration procedure, for example.

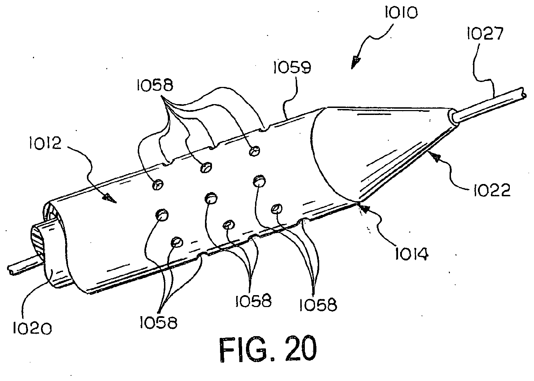

Figure 19 shows the distal end 1022 of the delivery system 1010 illustrated in Figure 1 according to another example. Repeated structure has the same last two digits of the reference numerals from the previous figures, preceded by a 10. For example, the delivery system is designated by the reference numeral 1010. The delivery system 1010 is disposed in a body vessel 1060. The tubular member 1012 includes a plurality of holes or hydration channels 1058 formed therein. The hydration channels 1058 extend radially outwardly from the device chamber 1030 formed in the dilator 1020 to an outer wall 1059 of the tubular member 1012 as shown in Figure 20. The hydration channels 1058 provide fluid communication between the device chamber 1030 and an environment surrounding the outer wall 1059 of the tubular member 1012. The environment surrounding the outer wall 1059 of the tubular member 1012 includes fluid 1062 being conveyed by the body vessel 1060. The hydration channels 1058 provide a communicative passageway that allows the fluid 1062 to hydrate the intraluminal medical device 1028. It is understood that more or fewer hydration channels 1058 can be used as desired. Although the plurality of hydration channels 1058 are shown as a plurality of annular arrays, other patterns of the hydration channels 1058 can be used. Any conventional method such as mechanical removal of material from the tubular member 1012 and other methods can be used to form the hydration channels 1058 in the tubular member 1012.

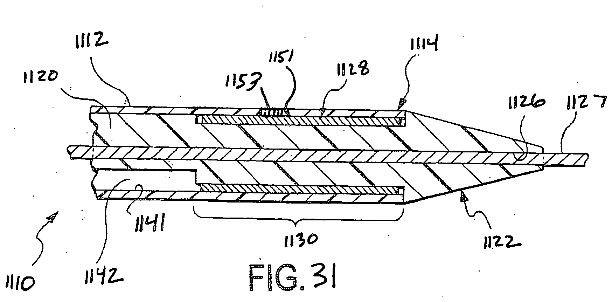

Figure 21 illustrates the distal end 1122 of a delivery system 1110 according to another example. Repeated structure is represented by the same two digit reference numerals from Figure 1, preceded by "11". For example, the delivery system is designated by the reference numeral 1110. In the illustrated embodiment, the tubular member 1112 defines a hydration channel 1151 that provides fluid communication between the device chamber 1130 and an environment external to the tubular member 1112. In this embodiment, a septum, such as resealable septum 1153, is disposed in the hydration channel 1151. Any suitable septum can be used. Resealable septums are considered advantageous because they can permit access to the device chamber 1130 by a hydrating device, such as a needle operably connected to a syringe, and seal upon retraction of the device to substantially prevent egress of a hydrating fluid introduced into the device chamber 1130 by the hydrating device. An example of a suitable septum is a resealable elastomeric septum known in the art and commonly used to provide such access to needles.

The delivery device 1110 can optionally include one or more additional hydration channels, such as hydration channel 1142 formed between the dilator 1120 and an adjacent wall 1141 of the tubular member 1112.

The intraluminal medical device 1128 of the delivery system 1110 illustrated in Figure 21 is hydrated by inserting a hydration fluid through the septum 1153 and into the device chamber 1130. This can be conducted using any suitable technique, such as by passing a portion of a hydration device, such as a needle connected to a syringe, through the septum 1153 and subsequently introducing the hydration fluid into the chamber 1130.

Delivery systems according to this exemplary embodiment are conveniently provided as kits that include the delivery system, a hydrating fluid, and a hydrating device. An intraluminal medical device can be disposed within the device chamber of the delivery device. A kit according to one exemplary embodiment includes a delivery device with an intraluminal device disposed in the device chamber and a syringe containing a hydrating fluid. In another exemplary embodiment, a kit includes a delivery device with a prosthetic valve disposed in the device chamber, and a syringe containing a hydrating fluid. The prosthetic valve includes a material that can be hydrated by the hydration fluid, such as an ECM. SIS is one exemplary material that can be hydrated. Suitable prosthetic valves are described in United States Patent 6,200,336 and 6,508,833 to Pavcnik et al. for a MULITPLE- SIDED INTRALUMINAL MEDICAL DEVICE.

In another exemplary embodiment, a kit includes a delivery device with a support frame disposed in the device chamber, a hydrating fluid, and a hydrating device. In one particular embodiment, the hydrating fluid contains a bioactive that can be advantageously associated with the intraluminal device.

Any suitable support frame can be used. The support frame can be balloon or self expandable, and can have any suitable configuration, including braided strands, helically wound strands, ring members, consecutively attached ring members, tube members, and frames cut from solid tubes. Also, suitable support frames can have a variety of sizes. The exact configuration and size chosen will depend on several factors, including the desired delivery technique, the nature of the vessel in which the device will be implanted, and the size of the vessel. The support frame can be sized so that the second, expanded configuration is larger in diameter that the inner diameter of the vessel in which the device will be implanted. This sizing can facilitate maintenance of the device in the vessel following implantation.

Examples of suitable support frames for use in the medical devices of the invention include those described in United States Patents 6,508,833 to Pavcnik et al. for a MULTIPLE- SIDED INTRALUMINA MEDICAL DEVICE; 6,464,720 to Boatman et al. for a RADIALLY EXPANDABLE STENT; 6,231,598 to Berry et al. for a RADIALLY EXPANDABLE STENT; 6,299,635 to Frantzen for a RADIALLY EXPANDABLE NON-AXIALLY CONTRACTING SURGICAL STENT; 4,580,568 to Gianturco for a PERCUTANEOUS ENDOVASCULAR STENT AND METHOD FOR INSERTION THEREOF; and published application for United States Patent 20010039450 to Pavcnik et al. for an IMPLANTABLE MEDICAL DEVICE.

If included, the hydrating fluid can comprise any suitable bioactive and the specific bioactive or bioactives chosen for any particular embodiment will depend on several considerations, including the condition being treated with use of the kit.

Examples of suitable bioactives include antithrombogenic agents, antiproliferative agents, and immunosuppressive agents. A wide range of other bioactives can be used, including heparin, covalent heparin, or another thrombin inhibitor, hirudin, hirulog, argatroban, D-phenylalanyl-L-poly-L-arginyl chloromethyl ketone, or another antithrombogenic agent, or mixtures thereof; urokinase, streptokinase, a tissue plasminogen activator, or another thrombolytic agent, or mixtures thereof; a fibrinolytic agent; a vasospasm inhibitor; a calcium channel blocker, a nitrate, nitric oxide, a nitric oxide promoter or another vasodilator; Hytrin.RTM. or other antihypertensive agents; an antimicrobial agent or antibiotic; aspirin, ticlopidine, a glycoprotein IIb/IIIa inhibitor or another inhibitor of surface glycoprotein receptors, or another antiplatelet agent; colchicine or another antimitotic, or another microtubule inhibitor, dimethyl sulfoxide (DMSO), a retinoid or another antisecretory agent; cytochalasin or another actin inhibitor; or a remodeling inhibitor; deoxyribonucleic acid, an antisense nucleotide or another agent for molecular genetic intervention; methotrexate or another antimetabolite or antiproliferative agent; tamoxifen citrate, Taxol.RTM. or the derivatives thereof, or other anti-cancer chemotherapeutic agents; dexamethasone, dexamethasone sodium phosphate, dexamethasone acetate or another dexamethasone derivative, or another anti-inflammatory steroid or non-steroidal anti-inflammatory agent; cyclosporin or another immunosuppressive agent; trapidal (a PDGF antagonist), angiopeptin (a growth hormone antagonist), angiogenin, a growth factor or an anti-growth factor antibody, or another growth factor antagonist; dopamine, bromocriptine mesylate, pergolide mesylate or another dopamine agonist; .sup.60 Co (5.3 year half life), .sup.192 Ir (73.8 days), .sup.32 P (14.3 days), .sup.111 In (68 hours), .sup.90 Y (64 hours), .sup.99m Tc (6 hours) or another radiotherapeutic agent; iodine-containing compounds, barium-containing compounds, gold, tantalum, platinum, tungsten or another heavy metal functioning as a radiopaque agent; a peptide, a protein, an enzyme, an extracellular matrix component, a cellular component or another biologic agent; captopril, enalapril or another angiotensin converting enzyme (ACE) inhibitor; ascorbic acid, alpha tocopherol, superoxide dismutase, deferoxamine, a 21-aminosteroid (lasaroid) or another free radical scavenger, iron chelator or antioxidant; a .sup.14 C-, .sup.3 H-, .sup.131 I-, .sup.32 P- or .sup.36 S-radiolabelled form or other radiolabelled form of any of the foregoing; estrogen or another sex hormone; AZT or other antipolymerases; acyclovir, famciclovir, rimantadine hydrochloride, ganciclovir sodium, Norvir, Crixivan, or other antiviral agents; 5-aminolevulinic acid, meta-tetrahydroxyphenylchlorin, hexadecafluoro zinc phthalocyanine, tetramethyl hematoporphyrin, rhodamine 123 or other photodynamic therapy agents; an IgG2 Kappa antibody against Pseudomonas aeruginosa exotoxin A and reactive with A431 epidermoid carcinoma cells, monoclonal antibody against the noradrenergic enzyme dopamine beta-hydroxylase conjugated to saporin or other antibody targeted therapy agents; gene therapy agents; and enalapril and other prodrugs; Proscar.RTM., Hytrin.RTM. or other agents for treating benign prostatic hyperplasia (BHP) or a mixture of any of these.

In some embodiments, the bioactive is used to inhibit restenosis, such as paclitaxel, rapamycin, and other bioactives able to inhibit restenosis of a body vessel. Also, the bioactive can comprise a bioactive capable of producing another bioactive that has a desired effect, such as a nitric oxide (NO) producing and/or releasing entity. NO may have one or more desired treatment effects, including the ability to inhibit restenosis of a body vessel. Furthermore, two or more bioactives can be used.

Assembly of the delivery system 110 can be accomplished using any suitable technique. In one exemplary method of assembly, the intraluminal medical device 128 is disposed in the device chamber 130 of the dilator 120. The dilator 120 is inserted into the tubular member 112 to be substantially concentric therewith. The dilator 120 is inserted until the intraluminal medical device 128, the dilator 120, and the tubular member 112 are in the configuration shown in Figure 3. The tubular member 112 and the dilator 120 cooperate to maintain proper positioning of the intraluminal medical device 128 in the delivery system 110. Typically, the wireguide 127 is inserted into the lumen 126 during insertion of the distal end 122 of the dilator 120 into the body vessel (not illustrated in Figure 3). This exemplary method can be applied to all embodiments illustrated herein, and the order of the steps is exemplary in nature and is not necessary, critical or intended to limit the invention in any way.

Use of the delivery system 110 as herein described applies to each of the embodiments shown. In all methods described herein, the order of the steps is exemplary in nature and is not necessary, critical or intended to limit the invention in any way. In use, the delivery system 110 delivers the intraluminal medical device 128 to a desired location within the body vessel. To deliver the intraluminal medical device 128, the wireguide 127 is placed in the body vessel of a patient by navigating a distal end of the wireguide 127 to a desired area of treatment. A proximal end of the wireguide 127 is left outside the body of the patient.

When it is desired to insert the delivery system 110 in the body vessel, the proximal end of the wireguide 127 is inserted into the lumen 126 of the dilator 120 at the distal end 122. The distal end 122 of the dilator 120 is caused to enter the body vessel along the wireguide 127 and is moved to the desired area of treatment. Typically, deployment of the intraluminal medical device 128 at a desired area of treatment can be accomplished by causing the intraluminal medical device 128 and the distal end 122 of the dilator 120 to be slidingly moved out of the tubular member 112 or by retracting the tubular member 112 to reveal the intraluminal medical device 128..

In certain situations, it is desirable to hydrate the intraluminal medical devices 128, 228, 328, 428, 528, 628, 728, 828, 928, 1028. Such situations can include those where the intraluminal medical devices 128, 228, 328, 428, 528, 628, 728, 828, 928, 1028 require hydration in order to facilitate the desired function. Devices including natural materials, such as ECM materials, including small intestine submucosa (SIS), can be hydrated to restore the material to a desired size, shape or configuration, or to prevent undesirable effects, such as formation of adhesions with the material and a body tissue and/or fluid. Also, some coatings and materials, such as hydragels, can be hydrated to effect swelling, release of a bioactive agent, or other desirable change. Hydration can also be used to lubricate an intraluminal medical device prior to implantation.

The hydration can occur prior to insertion or delivery of the intraluminal medical devices 128, 228, 328, 428, 528, 628, 728, 828, 928, 1028, during insertion, or during delivery, as desired. The hydration process is being described for each of the embodiments for clarity. The order of the steps for each embodiment is exemplary in nature and is not necessary or critical or intended to limit the invention in any way. A hydrating fluid is provided from a source of hydrating fluid which may include a container, the body vessel, and the like, for example. The hydrating fluid can be any suitable fluid and the specific fluid chosen in any particular application will depend of several considerations, including the nature of the medical device being hydrated and the nature of the medical procedure being conducted. Non-limiting examples of suitable hydrating fluids include water, physiological saline, body fluids such as blood and plasma, and other suitable fluids.

For the embodiment illustrated in Figures 3 and 4, hydration is accomplished by the following steps. The delivery system 110 is provided having the structure shown and described in Figures 3 and 4. A hydrating fluid (not shown) is introduced into the hydration channel 140 at the proximal end 124 of the dilator 120. The hydrating fluid can be introduced into the hydration channel 140 by any conventional means such as using a syringe connected to the connector 118, for example. Various hydrating fluids can be used, as described above, such as a saline solution and blood, for example. The hydrating fluid is caused to flow through the hydrating channel 140 to the device chamber 130. Once in the device chamber 130, the hydrating fluid communicates with the intraluminal medical device 128 to provide hydration thereof. The hydrating fluid can then be removed or left to remain in the hydration channel 140 and the device chamber 130 as desired.

For the embodiment illustrated in Figures 5 and 6, hydration is accomplished by the following steps. The delivery system 210 is provided having the structure shown and described in Figures 5 and 6. A hydrating fluid (not shown) is introduced into at least one of the hydration channels 242 at the proximal end 224 of the dilator 220,. The hydrating fluid can be introduced into the hydration channels 242 by any conventional means such as using a syringe connected to the connector 218, for example. Various hydrating fluids can be used as desired such as a saline solution and blood, for example. The hydrating fluid is caused to flow through the hydrating channels 242 to the device chamber 230. Once in the device chamber 230, the hydrating fluid communicates with the intraluminal medical device 228 to provide hydration thereto, illustrated by 278. The hydrating fluid can then be removed or left to remain in the hydration channels 242 and the device chamber 230 as desired. By being spaced apart as shown in Figure 6, the hydration channels 242 allow for a distributed flow of the hydration fluid into the device chamber 230 to facilitate complete hydration of the intraluminal medical device 228.

In the embodiment illustrated in Figures 7 and 8, hydration is accomplished by the following steps. The delivery system 310 is provided having the structure shown and described in Figures 7 and 8. A hydrating fluid (not shown) is introduced into at least one of the hydration channels 344 at the proximal end 324 of the dilator 320. The hydrating fluid can be introduced into the hydration channels 344 by any conventional means such as using a syringe connected to the connector 318, for example. Various hydrating fluids can be used as desired such as a saline solution and blood, for example. The hydrating fluid is caused to flow through the hydrating channels 344 to the device chamber 330. Once in the device chamber 330, the hydrating fluid communicates with the intraluminal medical device 328 to provide hydration thereto. The hydrating fluid can then be removed or left to remain in the hydration channels 344 and the device chamber 330 as desired. By being spaced apart as shown in Figure 8, the hydration channels 344 allow for a distributed flow of the hydration fluid into the device chamber 330 to facilitate complete hydration of the intraluminal medical device 328.

With respect to the embodiment illustrated in Figures 9 and 10, hydration is accomplished by the following steps. The delivery system 410 is provided having the structure shown and described in Figures 9 and 10. A hydrating fluid (not shown) is introduced into the hydration channel 446 at the proximal end 424 of the dilator 420. The hydrating fluid can be introduced into the hydration channel 446 by any conventional means such as using a syringe connected to the connector 418, for example. Various hydrating fluids can be used as desired such as a saline solution and blood, for example. The hydrating fluid is caused to flow through the hydrating channel 446 to the device chamber 430. Once in the device chamber 430, the hydrating fluid communicates with the intraluminal medical device 428 to provide hydration thereof The hydrating fluid can then be removed or left to remain in the hydration channel 446 and the device chamber 430 as desired. In the embodiment shown, the hydrating fluid is also in communication with the lumen 426 formed in the dilator 420. Desirable results such as the hydrating fluid facilitating a sliding of the wireguide 427 in the lumen 426, for example, are achieved with this configuration.

In the embodiment illustrated in Figures 11 and 12, hydration is accomplished by the following steps. The delivery system 510 is provided having the structure shown and described in Figures 11 and 12. A hydrating fluid (not shown) is introduced into at least one of the hydration channels 548 at the proximal end 524 of the dilator 520. The hydrating fluid can be introduced into the hydration channels 548 by any conventional means such as using a syringe connected to the connector 518, for example. Various hydrating fluids can be used as desired such as a saline solution and blood, for example. The hydrating fluid is caused to flow through the hydrating channels 548 to the device chamber 530. Once in the device chamber 530, the hydrating fluid communicates with the intraluminal medical device 528 to provide hydration thereto. The hydrating fluid can then be removed or left to remain in the hydration channels 548 and the device chamber 530 as desired. By providing the annular array of hydration channels 548, the hydration channels 548 allow for a distributed flow of the hydration fluid into the device chamber 530 to facilitate complete hydration of the intraluminal medical device 528.

For the embodiment illustrated in Figures 13 and 14, hydration is accomplished by the following steps. The delivery system 610 is provided having the structure shown and described in Figures 13 and 14 A hydrating fluid (not shown) is introduced into the hydration channel 650 at the proximal end 624 of the dilator 620. The hydrating fluid can be introduced into the hydration channel 650 by any conventional means such as using a syringe connected to the connector 618, for example. Various hydrating fluids can be used as desired such as a saline solution and blood, for example. The hydrating fluid is caused to flow through the hydrating channel 650 to the device chamber 630. Once in the device chamber 630, the hydrating fluid communicates with the intraluminal medical device 628 to provide hydration thereof. The hydrating fluid can then be removed or left to remain in the hydration channel 650 and the device chamber 630 as desired.

With respect to the embodiment illustrated in Figures 15 and 16, hydration is accomplished by the following steps. The delivery system 710 is provided having the structure shown and described in Figures 15 and 16. A hydrating fluid (not shown) is introduced into the hydration channel 752 at the distal end 722 of the dilator 720. The hydrating fluid can be introduced into the hydration channel 752 by any conventional means such as by capillary action from a container containing the hydrating fluid (not shown) or the blood stream in the body vessel, for example. Various hydrating fluids can be used as desired such as a saline solution and blood, for example. The hydrating fluid is caused to flow through the hydrating channel 752 to the device chamber 730. Once in the device chamber 730, the hydrating fluid communicates with the intraluminal medical device 728 to provide hydration thereof. The hydrating fluid can then be removed or left to remain in the hydration channel 752 and the device chamber 730 as desired. By being provided substantially the same size as the lumen 726, the hydration channel 752 can function as a second lumen to receive a second wireguide or other device as desired.

In the embodiment illustrated in Figure 17, hydration is accomplished by the following steps. The delivery system 810 is provided having the structure shown and described in Figure 17. A hydrating fluid (not shown) is introduced into the lumen 826 formed in the dilator 820, and eventually the hydrating channels 854. The hydrating fluid can be introduced at the proximal end 824 of the dilator 820 or the distal end 822 of the dilator 820, as desired. The hydrating fluid can be introduced into the lumen 826 by any conventional means such as using a syringe connected to the connector 818 to "push" the hydrating fluid through the lumen 826 from the proximal end 824 or "draw" the hydrating fluid into the distal end 822 from a container containing the hydrating fluid (not shown) or from a lumen of a body vessel, for example. The hydrating fluid can also be drawn into the lumen by capillary action. Various hydrating fluids can be used as desired such as a saline solution and blood, for example. The hydrating fluid is caused to flow through the lumen 826 and through the hydrating channels 854 to the device chamber 830. Once in the device chamber 830, the hydrating fluid communicates with the intraluminal medical device 828 to provide hydration thereto. The hydrating fluid can then be removed or left to remain in the hydration channels 854 and the device chamber 830 as desired. By providing the plurality of annular arrays of hydration channels 854, the hydration channels 854 allow for a distributed flow of the hydration fluid into the device chamber 830 to facilitate complete hydration of the intraluminal medical device 828.

For the embodiment illustrated in Figure 18, hydration is accomplished by the following steps. The delivery system 910 is provided having the structure shown and described in Figure 18. The plug 956 is provided and inserted into the lumen 926 at the distal end 922 of the dilator 920. A hydrating fluid (not shown) is introduced into the lumen 926 formed in the dilator 920, and eventually the hydrating channels 954. In this embodiment, the hydrating fluid is introduced at the proximal end 924 of the dilator 920. The hydrating fluid can be introduced into the lumen 926 by any conventional means such as using a syringe connected to the connector 918 or by capillary action, for example. Various hydrating fluids can be used as desired such as a saline solution and blood, for example. The hydrating fluid is caused to flow through the lumen 926 and through the hydrating channels 954 to the device chamber 930. Once in the device chamber 930, the hydrating fluid communicates with the intraluminal medical device 928 to provide hydration thereto. The hydrating fluid can then be removed or left to remain in the hydration channels 954 and the device chamber 930 as desired. Removal of the hydrating fluid can include removal of the plug 956. By providing the plurality of annular arrays of hydration channels 954, the hydration channels 954 allow for a distributed flow of the hydration fluid into the device chamber 930 to facilitate complete hydration of the intraluminal medical device 928.

In the embodiment of the invention illustrated in Figures 19 and 20, hydration is accomplished by the following steps. The delivery system 1010 is provided having the structure shown and described in Figures 19 and 20. A hydrating fluid (not shown) is introduced into the hydration channels 1058 at the distal end 1014 of the tubular member 1012. The hydrating fluid can be introduced into the hydration channels 1058 by any conventional means such as by capillary action from a container containing the hydration fluid (not shown) or a fluid 1062 in the body vessel 1060, for example. Various hydrating fluids can be used as desired such as a saline solution and blood, for example. The hydrating fluid is caused to flow through the hydrating channels 1058 to the device chamber 1030. Once in the device chamber 1030, the hydrating fluid communicates with the intraluminal medical device 1028 to provide hydration thereto. The hydrating fluid can then be removed or left to remain in the hydration channels 1058 and the device chamber 1030 as desired. By providing the plurality of annular arrays of hydration channels 1058, the hydration channels 1058 allow for a distributed flow of the hydration fluid into the device chamber 1030 to facilitate complete hydration of the intraluminal medical device 1028.

In embodiments in which one or more hydration channels are formed in a distal end of a delivery device and communicate with an environment external to the distal end, such as the embodiments illustrated in Figures 15, 17, 18, and 19, hydration can be performed by inserting a portion of or the entire distal end of the device into the hydrating fluid. For example, the distal end can be dipped into a hydrating fluid within a container prior to insertion into a body vessel.

From the foregoing description, one ordinarily skilled in the art can easily ascertain the essential characteristics of this invention and, without departing from the scope thereof, can make various changes and modifications that are within the scope of the invention as set forth in the appended claims to adapt it to various usages and conditions.

## Claims

1. An intraluminal medical device delivery system, comprising:
an elongate tubular member (812) having a distal end adapted for insertion into a body vessel;
a dilator (820) disposed in said tubular member, said dilator having a lumen formed (826) in an axial direction therein and a distal end adapted for insertion into the body vessel, said tubular member and said dilator having a device chamber (830) formed therebetween; and
an intraluminal medical device (828) disposed in the device chamber; **characterised by**
a plurality of hydration channels (854) extending radially outward from the lumen to the device chamber to provide fluid communication between the device chamber and the lumen, to facilitate hydration of said intraluminal medical device.

2. The delivery system according to claim 1, wherein the distal end of the dilator defines a distal opening in fluid communication with said hydration channels.

3. The delivery system according to claim 2, further comprising a removeable plug disposed in the distal opening in the dilator.

4. The delivery system according to any preceding claim, wherein the plurality of hydration channels are formed as a plurality of annular arrays.

5. The delivery system according to any preceding claim, wherein the plurality of hydration channels are arranged in a helical pattern about the dilator.

6. The delivery system according any preceding claim, wherein the intraluminal medical device comprises a prosthetic valve.

## Patentansprüche

1. Intraluminales Abgabesystem für ein Medizinprodukt, umfassend:
ein langgestrecktes röhrenförmiges Element (812) mit einem distalen Ende zur Einführung in ein Körpergefäß;
einen in dem röhrenförmigen Element angeordneten Dilatator (820), wobei der Dilatator ein in einer axialen Richtung darin geformtes Lumen (826) und ein distales Ende zur Einführung in das Körpergefäß aufweist, wobei das röhrenförmige Element und der Dilatator eine dazwischen geformte Medizinproduktkammer (830) aufweisen; und
ein in der Medizinproduktkammer angeordnetes intraluminales Medizinprodukt (828), **gekennzeichnet durch**
eine Vielzahl von Hydratationskanälen (854), die sich von dem Lumen radial nach außen zu der Medizinproduktkammer erstrecken, um eine Fluidverbindung zwischen der Medizinproduktkammer und dem Lumen zur Erleichterung der Hydratation des intraluminalen Medizinprodukts bereitzustellen.

2. Abgabesystem nach Anspruch 1, worin das distale Ende des Dilatators eine distale Öffnung definiert, die mit den Hydratationskanälen in Fluidverbindung steht.

3. Abgabesystem nach Anspruch 2, ferner einen entfernbaren Stopfen umfassend, der in der distalen Öffnung in dem Dilatator angeordnet ist.

4. Abgabesystem nach einem der vorhergehenden Ansprüche, worin die Vielzahl von Hydratationskanälen als eine Vielzahl von ringförmigen Anordnungen geformt ist.

5. Abgabesystem nach einem der vorhergehenden Ansprüche, worin die Vielzahl von Hydratationskanälen in einem spiralförmigen Muster um den Dilatator angeordnet ist.

6. Abgabesystem nach einem der vorhergehenden Ansprüche, worin das intraluminale Medizinprodukt eine Klappenprothese umfasst.

## Revendications

1. Système de distribution d'un dispositif médical intraluminal, comprenant :
un organe tubulaire allongé (812) ayant une extrémité distale prévue pour être insérée dans un vaisseau corporel ;
un dilatateur (820) disposé dans ledit organe tubulaire, ledit dilatateur ayant une lumière (826) formée dans une direction axiale dans celui-ci et une extrémité distale prévue pour être insérée dans le vaisseau corporel, ledit organe tubulaire et ledit dilatateur ayant une chambre de dispositif (830) formée entre eux ; et
un dispositif médical intraluminal (828) disposé dans la chambre de dispositif ;
**caractérisé par**
une pluralité de canaux d'hydratation (854) s'étendant radialement vers l'extérieur depuis la lumière jusqu'à la chambre de dispositif afin d'assurer une communication fluidique entre la chambre de dispositif et la lumière, pour faciliter l'hydratation dudit dispositif médical intraluminal.

2. Système de distribution selon la revendication 1, dans lequel l'extrémité distale du dilatateur définit une ouverture distale en communication fluidique avec lesdits canaux d'hydratation.

3. Système de distribution selon la revendication 2, comprenant en outre un bouchon amovible disposé dans l'ouverture distale dans le dilatateur.

4. Système de distribution selon l'une quelconque des revendications précédentes, dans lequel la pluralité de canaux d'hydratation sont formés en tant que pluralité d'ensembles annulaires.

5. Système de distribution selon l'une quelconque des revendications précédentes, dans lequel la pluralité de canaux d'hydratation sont disposés suivant un motif hélicoïdal autour du dilatateur.

6. Système de distribution selon l'une quelconque des revendications précédentes, dans lequel le dispositif médical intraluminal comprend une valvule prothétique.
